Europäisches Patentamt

(19) European Patent Office

Office européen des brevets

(11) Publication number: **0 077 604**
**B1**

(12) **EUROPEAN PATENT SPECIFICATION**

(45) Date of publication of patent specification: **04.06.86**

(21) Application number: **82304504.2**

(22) Date of filing: **26.08.82**

(51) Int. Cl.⁴: **A 61 M 5/00,** A 61 M 5/14,
A 61 J 1/00

(54) **Cartridge for use in administering a dry medicine.**

(30) Priority: **26.08.81 US 296335**

(43) Date of publication of application:
**27.04.83 Bulletin 83/17**

(45) Publication of the grant of the patent:
**04.06.86 Bulletin 86/23**

(84) Designated Contracting States:
**AT BE CH DE FR GB IT LI LU NL SE**

(56) References cited:
**DE-A-2 354 575**
**FR-A-1 122 616**
**GB-A-1 461 161**
**GB-A-2 059 776**
**US-A-3 976 068**
**US-A-4 233 973**

(73) Proprietor: **ELI LILLY AND COMPANY**
**307, East McCarty Street**
**Indianapolis Indiana 46285 (US)**

(72) Inventor: **Harris, Dale Carvin**
**Box 13B R.R. 1**
**Fairland Indiana 46126 (US)**
Inventor: **Hargrove, William Walter**
**4715 Andover Square**
**Indianapolis Indiana 46226 (US)**

(74) Representative: **Crowther, Terence Roger et al**
**Erl Wood Manor**
**Windlesham Surrey GU20 6PH (GB)**

## Description

This invention relates to a cartridge for use in administering a dry medicine intravenously.

US—A—4,233,973 discloses an intravenous infusion set comprising a container for a powdery drug, the intravenous liquid being drawn into and expelled from the container by a piston-cylinder pumping arrangement. No provision is made to avoid undissolved drug being carried by the intravenous liquid into the patient.

GB—A—1,461,161 discloses a method of preparing a ready-for-use bottle of infusion solution. A solid drug from a squeezable container is dissolved in the solution by connecting the container to the solution bottle, the solution being brought into contact with the drug by successive squeezing and relaxing of the container. The container is provided with a barrier to avoid transfer of undissolved drug to the bottle.

The invention specifically provides a cartridge for storing a dosage of a pharmaceutical product in dry form, and for eventual use with an intravenous administration set to facilitate intravenous infusion of the dosage in solution in a suitable liquid, the intravenous administration set having a supply of the liquid connected to it during use and having connecting means for use in forming a liquid-communicating connection between the cartridge and the intravenous administration set, the cartridge comprising: a liquid-tight container for holding the dosage of dry pharmaceutical product; connecting means communicating with the container and adapted to mate with the connecting means of the intravenous administration set to form the liquid-communicating connection between the intravenous administration set and the container; characterised in that the container is provided with barrier means disposed inside the container for preventing solid particles larger than a predetermined size that could potentially cause irritation of veins from passing out of the container, the barrier means being pervious to liquids and solutions so that the liquid from the intravenous administration set can contact and dissolve the pharmaceutical product and the resulting solution of the pharmaceutical product can pass through the barrier means and through the communicating connection to the intravenous administration set for infusion, and in that the container is provided with an air vent to facilitate passage of liquid into the container from the intravenous administration set, the air vent including an air-pervious liquid barrier to prevent escape of liquid through the air vent.

Medical treatment frequently requires the administration of fluids and medication solutions intravenously. Such fluids can include saline and dextrose solutions and other solutions to correct imbalances in body chemistry and medication solutions to treat disease. Such solutions are frequently available in commercial aseptic solution containers that are adapted to be punctured at one end and be hung from the other end so that their liquid contents may be removed and infused in the vein of a patient.

In effecting such treatment, an intravenous administration set, commonly referred to as IV set, is used to puncture the closure of the solution container and to conduct the liquid material from the solution container to a hypodermic needle for injection into the veins of the patient. Included in such IV sets are a transparent drip chamber having a conduit-forming spike at one end to perforate and enter the solution container, a transparent, flexible, plastic tube attached at the other end of the drip chamber, a regulating clamp providing means to control the flow of liquid through the passageway of the plastic tube, one or more Y injection sites to provide means to attach other medicament dispensers, and a termination adapted to accept a hypodermic needle. Such IV sets may include a vacuum pump adapted for insertion and operation in pump-operating apparatus. The use of the IV sets requires aseptic techniques, and the IV set is protected against contamination from handling at the point of attachments to the solution container and the hypodermic needle by protective end caps. In the administration of solutions and medication intravenously, liquid flow may be regulated through adjustment of the regulating clamp (or any volume pump that may be in use). Frequently, liquid medications are added to other liquid solutions by attaching the source of liquid medication at a Y injection site, preferably upstream of any pump that may be in use.

Certain medicines are manufactured and packaged in dry form because their stability is impaired in liquid solution. Antibiotics are frequently manufactured and packaged for storage and shipment in dry form because of their instability in liquid solution.

Where dry medicines have been administered intravenously in the past, liquid solutions of the dry medicines have had to be prepared shortly before their administration commences. In hospital treatment, this has heretofore meant that the liquid solutions have had to be prepared from dry medicines at the hospital pharmacy for delivery to those personnel responsible for their administration. Liquid solutions of many dry medicines have required careful preparation to avoid injection of small particles of medicine into the veins of a patient because such particles of the medicine will "burn" or irritate the patient's veins, causing discomfort.

A principal object of the present invention is to provide an economical and easily used means to package an individual dosage of dry pharmaceutical product so that the pharmaceutical product can be stored safely until use, and can then be placed in solution at the patient's bedside rather than in the pharmacy.

Further features and advantages of the invention will be apparent from the following drawings and descriptions.

Fig. 1 is a drawing of a dry medicine, intravenous administration system of this invention;

Fig. 2 is a cross-sectional view of the cartridge-like, dry-medicine package and container that is shown in Fig. 1;

Fig. 3 is a cross-sectional view of another embodiment of the cartridge-like, dry-medicine package and container of this invention and the corresponding receptacle of the system;

Fig. 4 is a cross-sectional view of still another embodiment of the cartridge-like, dry medicine package and container of this invention;

Fig. 5 is a cross-sectional view of a receptacle for use with the package and cartridge of Fig. 4; and

Fig. 6 is a partial view of an IV administration system with the cartridge and receptacle of Figs. 4 and 5.

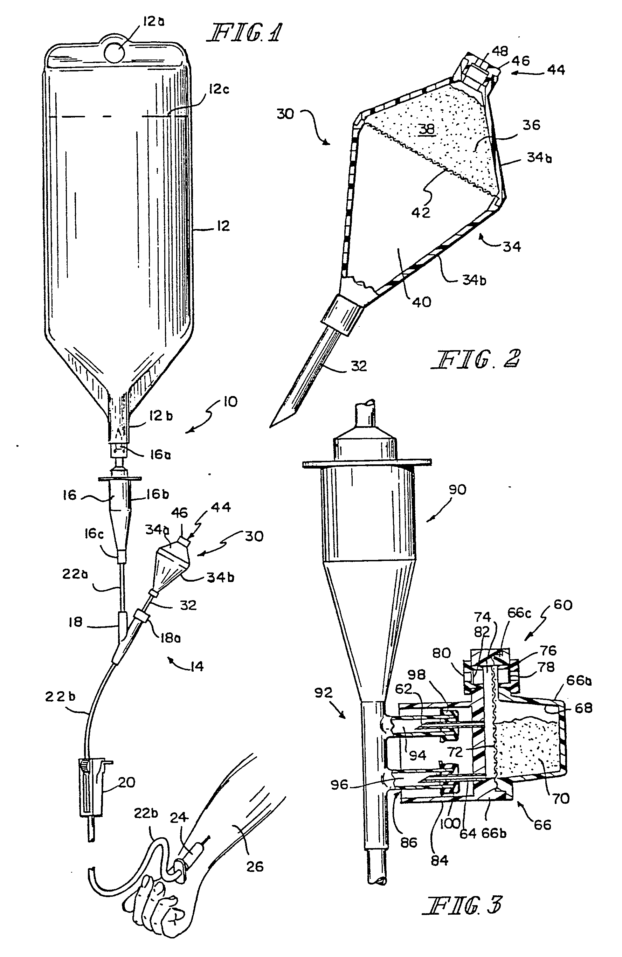

The intravenous administration system 10 shown in Fig. 1, includes a source of liquid diluent 12 and an IV administration set 14 including a drip chamber 16 with an adjacent attachment connecting means 18, such as a standard Y injection site, and a regulating clamp 20, all interconnected by means 22a and 22b, forming a passageway between the drip chamber 16 and a hypodermic needle 24 which is shown inserted into the vein of a patient to deliver liquid from the system into the patient.

The source of liquid 12 may be a solution container, for example, of the type manufactured by Travenol Laboratories, Inc., of Deerfield, Illinois 60015, and sold under the registered trademark "Viaflex" as a single-dose container. Such containers are clear, flexible plastic provided with an opening 12a at one end so that the source of liquid 12 may be suspended above the patient and a conduit-like closure 12b at the other end adapted for perforation and sealing engagement with the IV set 14.

The IV set 14 includes components of the type typically distributed by Ivac Corporation of San Diego, California 92121. In such a set, the drip chamber 16 is provided at one end with a spike-like conduit means 16a adapted to perforate and enter and provide a sealed engagement at its periphery with the conduit-like closure 12b of solution container 12. The central portion 16b of the drip chamber 16 is formed from transparent plastic to permit visual monitoring of the rate at which liquid is flowing from the source of liquid 12 into the veins of the patient 26. The downstream end 16c of the drip chamber 16 is attached to a transparent, flexible, plastic tube 22a to carry the liquid from the drip chamber 16. The Y injection site 18 provides means to attach and connect an additional source of medicine to the system. The Y injection site provides a sterile access to the passageway of the system which is closed by a standard rubber IV stopper 18a.

The flow of liquid from the source 12 is controllable through use of the regulating clamp 20. Such clamps can include a serrated roller adapted to engage the outer surface of flexible plastic tube 22b and to pinch, to a variable degree, and thereby impose a restriction upon the passageway of the tubing, controlling the rate at which liquid from source 12 passes the restriction formed in the tubing 22b by the regulating clamp 20.

In accordance with the invention the system shown in Fig. 1 includes a cartridge or package 30 for dry medicine 38. The dry medicine may be in powdered form or may be a single body of medicine, which, for example, may comprise a compressed mass of particulate medicine. The cartridge 30 may be added to the system 10 at the Y injection site, thereby permitting the dry medicine packaged in container 34 to be administered in solution in the liquid from source 12, as will be described in greater detail hereinafter.

Fig. 2 shows a partial cross-sectional view of the cartridge 30, and its operation in the system may be determined by reference to Fig. 1 and Fig. 2. The cartridge 30 includes a perforating conduit connecting means 32, which may be a standard hypodermic needle or other sharpened tubular conduit specifically manufactured as part of the cartridge 30. The cartridge 30 includes a container 34 for dry medicine 38. The container is preferably divided into a first cell 36, containing the dry medicine, and a second cell 40, as will be explained. A liquid-pervious barrier 42 is disposed in the container 34 between the dry, particulate medicine and the perforating conduit connecting means 32 to prevent particles of medicine from entering the IV system 14. The barrier 42 may define a wall separating the first cell 36 from the second cell 40. The package 30 is provided with an air vent 44 at the end of the cartridge opposite perforating conduit connecting means 32. The barrier 42 is pervious to air as well as liquid in cartridge 30 to permit air in the second cell 40 to reach and escape through air vent 44. Air vent 44 is formed by an opening 46 in the container 34 that is closed by an air-pervious, liquid barrier 48. An example of such an air-pervious, liquid barrier is a fluoroplastic material sold by the "Fluoro Techniques" Corporation under their designation M8A 2000. To avoid contamination of medicine within cartridge 30, the air vent 44 may be recessed as shown or provided with an additional air-pervious outer shield to prevent contact with the air-pervious liquid barrier 48 as cartridge 30 is handled. The container 34 is preferably a molded transparent thermoplastic material so that the interior of the cartridge 30 may be monitored visually in use.

The barrier 42 in the container may be any material which will transmit liquid but not particles of dry medicine having a size sufficient to reach and irritate the patient's veins. Such simple materials as common laboratory filter paper may function as a barrier in combination with a downstream filter to prevent paper fibers from being infused in the patient. Selection of the barrier material and its porosity is a major consideration in designing the system to provide different rates of drug administration. Another major consideration is the rate of solubility of the dry medicine in the liquid of the system. Where the dry medicine may pass rapidly into solution in the liquid of the system, selection of relatively non-porous barrier material will permit the rate of administration of the soluble medicine to be slowed and controlled. Where the dry medicine passes into solution very slowly (for example, where a single body of dry medicine is relatively insoluble in the liquid), a porous barrier material may be selected having pore sizes only sufficiently small to prevent the passage of medicine particles that will burn or irritate a patient's veins. Thus, the cartridge permits control of the rate of administration of many very soluble dry medicines and of dry medicines whose rates of solubility may not be controlled. The cartridge also permits the bedside administration of other dry medicines at rates controlled by their rates of solubility.

Such barrier materials may include non-particulate-forming plastic filter materials, such as those manufactured by Millipore Filter Corporation and Gelman Corporation. These materials are available in a variety of porosities. For example, one such material is sold by Millipore Filter Corporation under the designation GSWP 025 00 GSO.45 micron and can be incorporated into the system to provide low rates of administration of soluble medicines. In some systems, the barrier 42 may be a very fine screen.

The walls of the container 34 may be of either glass or another transparent thermoplastic material capable of aseptic treatment to insure sterile intravenous administration of the dry medicine.

A cartridge 30 such as that shown in Fig. 2 operates as follows in IV systems of the type shown in Fig. 1. The cartridge 30 is added to the system 10 of Fig. 1 by pressing its perforating conduit means 32 through the rubber IV stopper 18a. Because the level of the liquid 12c in the source of liquid 12 is higher than the container 34, fluid pressure provides a flow of liquid from the source 12 downwardly through the drip chamber 16, the flexible tubing 22a, the Y injection site 18, and upwardly through perforating conduit means 32 into the container 34. Because barrier 42 is pervious to air and liquid, liquid flows into compartment 36 and mingles with the dry medicine 38. The first cell 36 is completely filled because the fluid pressure will expel air or any other gaseous material within the container 34 outwardly through the air-pervious, liquid barrier 48 and air vent opening 46 formed in the container. The dry medicine 38 is thus exposed to the liquid of the system and is placed in solution. The liquid solution of dry medicine flows through the barrier 42 and outwardly through the perforating conduit means 32 of the package and into the Y injection site 18 and the flexible tube 22b to join the regulated flow of liquid through hypodermic needle 24 into the vein of the patient. The container 34 is maintained full of liquid because it remains below the level 21c of liquid in the system.

In the system of Figs. 1 and 2, therefore, the perforating conduit means 32 provides a two-way flow of liquid. At higher rates of administration, the higher specific gravity of the medicine solution can provide a significant outward flow under the influence of gravity, and as the medicine solution flows downwardly and outwardly of the cartridge 30, it is replaced by an upward flow of a less dense liquid from source 12, thus keeping the cartridge 30 full of liquid as part of the system. The rate of flow of medicine solution from cartridge 30 can be determined for very soluble medicines through selection of the porosity of barrier 42 and the diameter of the conduit in perforating conduit means 32. For example, where the barrier 42 has the porosity of laboratory filter paper, and the conduit of perforating conduit means 32 has a size at least that of a 16-gauge hypodermic needle, solutions of very soluble medicine can be administered from one gram doses within a time span of about two and one-half hours.

By reducing the porosity of the barrier 42, longer administration intervals may be obtained, and by increasing the porosity of the barrier, shorter administration intervals may be obtained. The rate of flow of medicine solution from the cartridge may thus be determined and generally controlled by design of the cartridge. The rate of flow of medicine solution from the cartridge is not affected to a significant degree by any regulating clamp or pump that may be downstream of the cartridge.

The cartridge 30 preferably provides a pocketless wall leading directly from the compartment for dry medicine to the conduit of the perforating conduit means. The container 34 of the cartridge 30 shown in Fig. 2, for example, provides a frustoconical wall 34b at an acute angle (e.g., 60°) with respect to the bore of perforating conduit means 32. The medicine solution formed in the container 34 can thus be substantially entirely drained from the container 34 and administered to the patient. The cartridge 30 in some circumstances may need to be supported or held in a generally vertical orientation for complete use of the container medicine.

Cartridges of the type shown in Fig. 2 may be manufactured by splitting the container 34 into two frustoconical wall portions, 34a forming the walls of a first cell or compartment, and 34b forming the walls of a second cell or compartment. Cartridges having a second cell 40 are preferable to avoid concentrating the flow of medicine solution into a congested area of the barrier 42 and to avoid blockage of the congested area with undissolved medicine and a resultant restriction of flow of medicine. Thus, as shown in Fig. 2, an air and liquid-pervious barrier 42 may be interposed between portions 34a and 34b of the container. Splitting the container 34 into two portions also permits the portion 34a forming ths first cell 36 to be varied in size for different medicine dosages while maintaining a standard configuration for the other portion 34b that forms the second cell 40 and carries the connecting means 32. The connecting means 32 may be a tubular steel conduit molded into the end of portion 34b opposite the barrier and sharpened to provide an exposed, needle-like extremity. The opening 46 and housing for the air-pervious liquid barrier 48 may be molded into the portion 34a of the container. Such a cartridge system can have a maximum diameter on the order of about one inch (2.54 cm) for a medicine dose of about one gram.

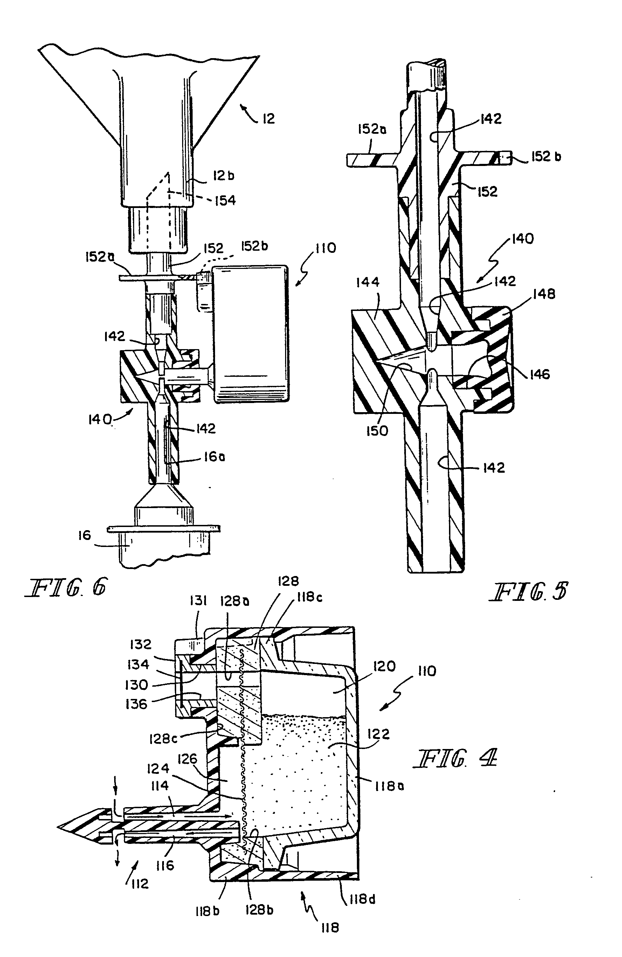

Another embodiment 60 of the cartridge of this invention is shown in Fig. 3. The cartridge of Fig. 3 incorporates two needles as its connecting means and two air vents. This embodiment is shown in Fig. 3 to use an especially designed drip chamber 90. The dual-needle system of Fig. 3 permits reduction of the size of the individual needles and, therefore, reduction of the size of the perforations created in the rubber IV stoppers of the system in use. Such a system can incorporate needles as perforating conduit means having a size comparable to 18- or 19-gauge hypodermic needles.

Like the cartridge 30 shown in Fig. 1, the cartridge 60 shown in Fig. 3 includes a perforating conduit means, needles 62 and 64, container 66 divided into a first cell or compartment 68 for dry powdered medicine 70, and a second cell 74. A liquid-pervious barrier 72 is interposed between the first cell 74 and the perforating conduit means 62, 64, which opens out of the second cell 74.

The container 66 may be molded from glass or other clear thermoplastic material that is capable of aseptic treatment, and can include two portions 66a and 66b. The first portion 66a forms the first cell or compartment 68 for dry medicine 70 that may be varied in size. The second portion 66b carries the perforated conduit means 62, 64 and forms a second cell or compartment 74 within the cartridge 60. The first package portion 66a and the second package portion 66b are adapted at their outer periphery 66c to provide a tongue and groove engagement to retain there between the barrier 72. The upper part of the first portion 66a of the cartridge is formed to provide an air vent opening 76 for the first cell 68 and to carry an air-pervious, liquid barrier 78. The upper part of the second portion 66b of the cartridge is formed to provide an air vent opening 80 for the second cell 74 and to carry an air-pervious, liquid barrier 82. Because both the first cell and second cell include air vents, the liquid-pervious barrier 72 need not be air-pervious. The air-pervious, liquid barriers 78 and 82 may be of the same type described above with respect to the cartridge of Fig. 2.

The second portion 66b of the container 66 can provide a standard element of the system with standard connecting means including a consistent spacing of the perforating conduit means 62, 64. The second portion may also include a projecting sleeve 84 surrounding and shielding the perforating conduit means 62, 64. Sleeve 84 provides an opening 86 that may be sealed to avoid contamination of the perforating conduit means 62, 64 and protect against injury of personnel by the perforating conduit means in handling the cartridge.

As shown in Fig. 3, a drip chamber 90 may be especially adapted in its lower portion 92 to provide connecting means comprised of two projecting openings 94 and 96 sealed with standard rubber IV stoppers 98 and 100. The projecting openings 94 and 96 to the system are spaced a distance corresponding to the spacing between the needles 62 and 64 of cartridge 60. Sleeve 84 may be of such size that it guides the needles of perforating conduit means 62 and 64 into engagement with stoppers 98 and 100 when the cartridge 60 is added to the system. Cartridge 60 thus may support itself as shown in Fig. 3. Although Fig. 3 indicates that lower portion 92 of drip chamber 90 is adapted to accept the cartridge 60, the system may be provided with a receptacle-like portion 92 which is separate from the drip chamber.

As shown in Fig. 3, the portions 66a and 66b of container 66 form a pocketless wall leading from the first chamber 68 to the bore of the lower perforating conduit means 64 to avoid the entrapment of medicine solution within the package and to provide the medicine substantially entirely to the patient.

For a dose of about one gram, such a container may be formed to provide a first compartment 68 having a depth on the order of 7/8 of an inch (2.22 cm) from the barrier 72 to the distal wall of portion 66a. The outer walls of portion 66a are preferably frustoconical, having an average diameter of about one inch (2.54 cm) adjacent the barrier 72 and decreasing to about 7/8 of an inch (2.22 cm) at the distal wall. The second portion 66b of the container, which can comprise a standard cartridge head, carries two 18- or 19-gauge needles 62, 64, each having an outside diameter of approximately .040 inch (0.1 cm) spaced typically somewhat over 5/8 of an inch (1.59 cm) apart. The needles 62, 64 project outwardly from portion 66b about 5/8 of an inch (1.59 cm) and are surrounded by a projecting sleeve 84 about 7/8 of an inch (2.22 cm) long. The outer sleeve 84 has, typically, the uniform thickness of about .060 inch (0.15 cm) and forms an opening approximately one inch (2.54 cm) in length and somewhat less than 3/8 of an inch (0.95 cm) in width that is centered upon the perforating conduit means 62, 64. The air vent openings 76 and 80 have an inside diameter on the order of one-quarter inch (0.64 cm). The overall length of such a one gram cartridge is about two inches (3.08 cm) and its overall diameter is about one and 3/4 inch (4.45 cm). The design and dimensions above may be varied for other configurations and dosages.

The embodiment of Fig. 3 will operate in a system the same as that shown in Fig. 1 in generally the same manner as the embodiment of Fig. 2. This can be visualized by substitution of the drip chamber 90 and cartridge 60 shown in Fig. 3 for the drip chamber 16 and cartridge 30 of Fig. 1. Since the container 60 in such a system will be entirely below the level 12c of the liquid in the source of liqud 12, upon addition of the cartridge 60 to the system and perforation of stoppers 98 and 100 by the needles 62, 64, the cartridge 60 will be filled entirely with liquid. Air from within compartments 68 and 74 will be expelled through openings 76 and 80, respectively. The dry medicine 70 in the first compartment 68 will be exposed to the liquid, and thereby placed in liquid solution. The medicine solution will flow through barrier 72 and outwardly through the bore of the lower perforating conduit means 64 and the opening 96 into the IV administration system. The container 66 will be maintained full of liquid by an inward flow through opening 94 of the system and the bore of upper perforating conduit means 62 into the interior of the cartridge. Such flow will continue until the medicine from within container 66 is substantially entirely delivered to the patient.

The receptacle 92 for the cartridge may have several configurations. It may provide an open passageway downwardly between projecting openings 94 and 96 so that liquid in the system can flow downwardly through the receptacle 92 in parallel with the flow through cartridge 60. The receptacle 92 may also be closed between projecting openings 94 and 96 so that the flow of liquid in the system is directed in its entirety through cartridge 60. Where there is no passageway in the receptacle 92 between projecting openings 94 and 96, a shunt comprising, for example, a second portion 66b of cartridge 60 that has been closed by a liquid-impervious solid wall in place of barrier 72, would normally be in place on the receptacle to permit flow in the absence of a cartridge. The receptacle may also include a valve (not shown) between the projecting openings 94 and 96 to permit selection of the above modes of operation of the system.

Still another embodiment 110 of the cartridge this invention is shown in Fig. 4. The cartridge of Fig. 4 incorporates a single needle 112 having two passages 114 and 116 as its perforating conduit connecting means.

The cartridge illustrated in Fig. 4 is designed for use with an IV set including an especially designed connecting means, comprising an adapter and receptacle 140, as shown in Fig. 5 and Fig. 6. When cartridge 110 with its multiple-passage perforating conduit connecting means 112 is inserted into receptacle 140 as shown in Fig. 6, the flow of liquid in the system is directed in its entirety through cartridge 110. The engagement of perforating conduit means 112 with receptacle 140 closes the passage 142 (Fig. 5) of the receptacle, and liquid enters the cartridge 110 through passage 114 and carries the dissolved medicine back to the IV administration system through passage 116.

Like cartridges 30 and 60, cartridge 110 includes a connecting means comprising a perforating conduit means 112, a container 118 divided into a first cell or compartment 120 for dry powdered medicine 122 and a second cell 126 by a liquid-pervious barrier 124 interposed between the dry medicine 122 and the perforating conduit means 112.

The container 118 may be molded from glass or other clear thermoplastic material that is capable of aseptic treatment, and can include two portions 118a and 118b. The first portion 118a forms the first cell or compartment 120 for dry medicine 122 that may be varied in size. The first portion 118a is preferably glass. The second portion 118b forms the perforated conduit means 112 and can form a second cell or compartment 126 within the cartridge 110. The second portion 118b may be ABS, rigid polyethylene or polypropylene.

The first container portion 118a and the second container portion 118b are adapted at their outer periphery 118c to provide a tongue and groove engagement. The barrier 124 is carried in a gasket 128 of elastomeric material, such as natural rubber. The gasket 128 is retained between the first portion 118a and second portion 118b upon assembly of the cartridge 110. Two openings 128a and 128b are formed in the gasket 128 and are closed by barrier 124 to the dry medicine 122 but not to air or liquid. Gasket 128 includes a sealing surface 128c between openings 128a and 128b that engages and seals against the portion 118b, thereby defining cell 126 between portion 118b and the barrier 124.

The upper part of the second portion 118b of the container is formed to provide an air vent opening 130 for the interior of the cartridge 110. An insert 132 that may be formed of a rigid moldable plastic carries an air-pervious, liquid barrier 134 in the air vent opening 130. Openings 130 and 128a permit the air or gas within compartments 126 and 120 to be expelled from within the cartridge 110 through the opening 136 of insert 132 and permit medicine compartment 120 to be completely filled with liquid solution. Because the air-pervious barrier 134 is liquid-impervious, the liquid will not escape the cartridge 110. The air-pervious, liquid barrier 134 may be of the same type described above with respect to the cartridge of Fig. 2.

As shown in Fig. 5, a receptacle 140 may have an especially adapted semi-rigid bottom portion 114 to provide a projecting opening 146 sealed with a standard rubber stopper 148. The portion 144 forms a socket 150 shaped to mate the perforating conduit connecting means 112 of the cartridge.

The receptacle 140 provides an adapter to permit the use of the dry medicine cartridge 110 with standard IV components. Fig. 6 shows the use of receptacle-adapter 140 and the manner in which its ends are adapted for insertion into such a standard IV system. In adapting a standard IV system to use cartridges 110, the receptacle 140 is first placed upon the spike-like conduit means 16a of the dry chamber 16 of a standard IV administration set, such as that shown in Fig. 1, by inserting the spike-like conduit means 16a into the bottom portion of passageway 142. The top portion 152 of receptacle 140 is rigid and forms a sharpened spike 154 about passageway 142. The spike 152 is used to perforate, enter, and provide a sealed engagement at its periphery with the conduit-like closure 12b of solution container 12. The upper portion 152 of the receptacle is provided with an extended surface 152a to assist the user in inserting the receptacle into the solution container 12. With the receptacle 140 in place, liquid from the solution container 12 may flow freely through passageway 142 of the receptacle.

Cartridge 110 and its dry medicine is placed in the IV system by perforating stopper 148 with the perforating conduit means 112 and seating it in socket 150 of the adapter 140. The cartridge 110 has a key 131 formed by portion 118b adjacent the air vent opening 130. The extended surface 152a of the receptacle 152 is formed with a slot 152b into which key 131 of the cartridge is inserted. Cartridge 110 may thus be supported as shown in Fig. 6. The perforating conduit means 112 when seated in socket 150 closes and substantially seals passage 142. Liquid above the socket 150 is then urged by gravity to flow through cartridge 110 by entering the upper passage 114 of the perforating conduit means 112 and leaving the lower passage 116. The container 110 can thus be filled with liquid solute and provide dry medicine in solution to the patient.

For a dose of about one gram, such a cartridge may be formed to provide a first compartment 120 having a depth on the order of 3/4 of an inch (1.91 cm) from the barrier 124 to the distal wall of portion 118a. The outer walls of portion 118a are preferably frustoconical, having an average diameter of about one inch (2.54 cm) adjacent the barrier 124 and decreasing to about 7/8 of an inch (2.22 cm) at the distal wall. The second portion 118b forms needle 112 having a length of about an inch (2.54 cm) and an outside diameter of approximately .190 inch (0.48 cm). The forward surface of needle 112 forms a cone with a solid angle of 40 1/4 at its apex to assist in perforation of stopper 148. The conduits 114 and 116 extend forwardly within the needle 112 and terminate adjacent the base of the conical forward end. Openings for the conduits 114 and 116 are formed by two slots across the needle 112 having a depth sufficient to intercept the conduits 114 and 116. The second portion 118b of the cartridge may be formed with a cylindrical skirt 118d that extends around and protects the first portion 118a from contact. The air vent opening 130 has an inside diameter on the order of one-quarter inch (0.64 cm). The overall length of such a one gram cartridge is about two inches (5.08 cm) and its overall diameter is about one and 1/2 inches (3.81 cm). The design and dimensions above may be varied for other configurations and dosages.

The embodiment of Fig. 4 will operate in a system in generally the same manner as the embodiments of Fig. 2 and Fig. 3. Since the cartridge 110 in such a system will be entirely below the level of the liquid in the source of liquid 12, upon addition of the cartridge 110 to the system and the perforation of stopper 148 by the needle 112, the cartridge 110 will be filled entirely with liquid. Air from within compartments 120 and 126 will be expelled through openings 128a and 136, respectively. The dry medicine 122 in compartment 120 will be exposed to the liquid, and thereby placed in liquid solution. The medicine solution will flow through barrier 124 and outwardly through the lower conduit 116 into the IV administration system. The container 118 will be maintained full of liquid by an inward flow through the upper conduit 114 into the interior of the cartridge. Such flow will continue until the medicine from within cartridge 110 is substantially entirely delivered to the patient.

The invention may be used, for example, to permit the treatment of diseases that require periodic high blood concentrations of antibiotic medicine, and permit such concentrations from dry antibiotic medicines at planned, periodic intervals. The invention may provide an opportunity to kill bacteria which require high antibiotic concentrations. Such a system may be particularly adapted for use with cephalothin sodium antibiotics such as those sold by Eli Lilly and Company under the trademark "Keflin" and may provide periodic high blood concentrations of the antibiotic by administering the medicine in solutions over short intervals. The system is not limited to antibiotics, but also provides means to deliver other dry medicines, for example, cardiovascular drugs and others, in a variety of dosages. The system is capable of design to provide many rates of administration. Since the medicine is administered directly from its package, it may be stored and used by hospital personnel without resort to the hospital pharmacist.

Thus, as has been shown in the foregoing description, the present invention provides an economical and easily used means to package an individual dosage of dry pharmaceutical product so that the pharmaceutical product can be safely stored until use, and can then be placed in solution at the patient's bedside and administered by infusion. The cartridge provided by the invention can be designed to administer different dry medicines over different time periods and can permit repeated administrations on a scheduled interval. Since the dry medicine does not have to be prepared for intravenous administration at or by a hospital pharmacy, it can be stored and used by personnel on a hospital floor.

Tve preferred embodiments of the invention described above are capable of many modifications. Changes in the construction and arrangement may be made without departing from the scope of the invention as disclosed in the following claims.

**Claims**

1. A cartridge (30, 60, 110) for storing a dosage of a pharmaceutical product (38, 70, 122) in dry form, and for eventual use with an intravenous administration set (14) to facilitate intravenous infusion of the dosage in solution in a suitable liquid (12), the intravenous administration set (14) having a supply of the liquid (12) connected to it during use and having connecting means (18, 98, 100, 140) for use in forming a liquid-communicating connection between the cartridge and the intravenous administration set, the cartridge comprising: a liquid-tight container (34, 66, 118) for holding the dosage of dry pharmaceutical product (38, 70, 122);

connecting means (32, 62, 64, 112) communicating with the container and adapted to mate with the connecting means (18, 98, 100, 140) of the intravenous administration set (14) to form the liquid-communicating connection between the intravenous administration set (14) and the container (34, 66, 118); characterised in that the container is provided with barrier means (42, 72, 124) disposed inside the container (34, 66, 118) for preventing solid particles larger than a predetermined size that could potentially cause irritation of veins from passing out of the container (34, 66, 118), the barrier means (42, 72, 124) being pervious to liquids and solutions so the liquid from the intravenous administration set (14) can contact and dissolve the pharmaceutical product (38, 70, 122) and the resulting solution of the pharmaceutical product can pass through the barrier means (42, 72, 124), and through the communicating connection to the intravenous administration set (14) for infusion, and in that the container (34, 66, 118) is provided with an air vent (44, 76, 80, 130) to facilitate passage of liquid into the container from the intravenous administration set, the air vent including an air-pervious liquid barrier (48, 78, 82, 134) to prevent escape of liquid through the air vent.

2. The cartridge (39, 60, 110) of claim 1 including a dosage of dry pharmaceutical product (38, 70, 122) disposed in the container (34, 66, 118).

3. For use with an intravenous administration set (14) having a connecting means (18, 98, 100, 140) that includes an elastomeric stopper (18a, 98, 100, 148), a cartridge (30, 60, 110) in accordance with claim 1 wherein the connecting means (32, 62, 64, 112) of the cartridge comprises a conduit having an internal channel that communicates at one end with the container and terminates in a port at the distal end of the conduit, the distal end of the conduit being adapted to penetrate the stopper of the administration set to form the communicating connection.

4. The cartridge (110) of claim 1 wherein the connecting means (112) of the cartridge comprises a conduit, (112) having a first internal channel (114) which communicates at one end with the container (118) and terminates at the distal end of the conduit in a first port for carrying liquid from the intravenous administration set to the container, and a second, separate internal channel (116) which communicates at one end with the container (118) and terminates at the distal end of the conduit in a second port for carrying the solution of pharmaceutical product (122) from the container (118) to the intravenous administration set, said cartridge (110) being for use with an intravenous administration set having connecting means (140) adapted to direct the liquid from the intravenous administration set to the first port and receive the solution from the second port after the liquid has passed through the container.

5. The cartridge of claim 1 wherein the connecting means (62, 64) of the cartridge (60) includes a plurality of conduits (62, 64), each of which has an internal channel that communicates at one end with the container (66) and terminates in a port at the distal end of the conduit.

**Patentansprüche**

1. Patrone (30, 60, 110) zur Lagerung einer Dosis eines Arzneimittels (38, 70, 122) in trockener Form und zum späteren Gebrauch in Verbindung mit einerm intravenösen Infusionsgerät (14) zwecks Erleichterung der intravenösen Infusion der in einer geeigneten Flüssigkeit (12) gelösten Dosis, wobei das intravenöse Infusionsgerät (14) beim Gebrauch mit einem die Flüssigkeit (12) enthaltenden Behältnis verbunden ist und Verbindungsmittel (18, 98, 100, 140) zur flüssigkeitsführenden Verbindung zwischen der Patrone und dem intravenösen Infusionsgerät aufweist und wobei die Patrone einen flüssigkeitsdichten Behälter (34, 66, 118) zur Aufnahme der Dosis des trockenen Arzneimittels (38, 70, 122) sowie Verbindungsmittel (32, 62, 64, 112) zum Anschließ dieses Behälters an die Verbindungsmittel (18, 98, 100, 140) des Infusionsgeräts (14) aufweist, welche die flüssigkeitsführende Verbindung zwischen dem Infusionsgerät (14) und dem Behälter (34, 66, 118) bilden, dadurch gekennzeichnet, daß im Inneren des Behälters (34, 66, 118) eine Sperre (42, 72, 124) vorgesehen ist, die einen Austritt von über einer vorbestimmten Größe liegenden Feststoffteilchen, welche zu Reizungen der Venen führen können, aus dem Behälter (34, 66, 188) verhindert, wobei diese Sperre (42, 72, 124) jedoch für Flüssigkeiten und Lösungen so durchlässig ist, daß die Flüssigkeit aus dem intravenösen Infusionsgerät (14) mit dem Arzneitmittel (38, 70, 122) in Kontakt treten und dieses lösen kann und daß die erhaltene Lösung des Arzneimittels die Sperre (42, 72, 124) und die flüssigkeitsführende Verbindung zum intravenösen Infusionsgerät (14) zwecks Infusion passieren kann, und daß der Behälter (34, 66, 118) eine den Zustrom von Flüssigkeit aus dem intravenösen Infusionsgerät in den Behälter erleichternde Entlüftungsöffnung (44, 76, 80, 130) aufweist, welche eine luftdurchlässige Flüssigkeitssperre (46, 78, 82, 134) enthält, die ein Entweichen von Flüssigkeit durch die Entlüftungsöffnung verhindert.

2. Patrone (30, 60, 110) nach Anspruch 1, dadurch gekennzeichnet, daß ihr Behälter (34, 66, 118) eine Dosis eines trockenen Arzneimittels (38, 70, 122) enthält.

3. Patrone (30, 60, 110) nach Anspruch 1 zum Gebrauch in Verbindung mit einem intravenösen Infusionsgerät (14), dessen Verbindungsmittel (18, 98, 100, 140) einen elastomeren Stopfen (18a, 98, 100, 148) aufweist, dadurch gekennzeichnet, daß das Verbindungsmittel (32, 62, 64, 112) der Patrone aus einer Leitung besteht, die einen Innenkanal aufweist, dessen eines Ende mit dem Behälter in Verbindung steht und dessen anderes Ende eine Durchgangsöffnung aufweist, die so ausgebildet ist, daß sie eine Durchstechen des Stopfens des Infusionsgerätes zwecks flüssigkeitsführender Verbindung ermöglicht.

4. Patrone (110) nach Anspruch 1, dadurch gekennzeichnet, daß das Verbindungsmittel (112) der Patrone aus einer Leitung (112) besteht, die einen ersten Innenkanal (114), dessen eines Ende mit dem Behälter (118) in Verbindung steht und dessen anderes Ende eine erste Durchgangsöffnung aufweist, durch welche Flüssigkeit aus dem intravenösen Infusionsgerät in den Behälter strömt, und die einen von ersten Innenkanal getrennten zweiten Innenkanal (116) aufweist, dessen eines Ende ebenfalls mit dem Behälter (118) in Verbindung steht und dessen anderes Ende eine zweite Durchgangsöffnung aufweist, durch welche die Lösung des Arzneimittels (122) vom Behälter (118) in das intravenöse Infusionsgerät strömt, und daß die Patrone (110) zum Gebrauch in Verbindung mit einem intravenösen Infusionsgerät bestimmt ist, dessen Verbindungsmittel (140) so ausgebildet ist, daß es die Flüssigkeit vom intravenösen Infusionsgerät über die erste Durchgangsöffnung zuführt und die Lösung nach dem Durchströmen des Behälters über die zweite Durchgangsöffnung abführt.

5. Patrone (60) nach Anspruch 1, dadurch gekennzeichnet, daß das Verbindungsmittel (62, 64) der Patrone aus mehreren Leitungen (62, 64) besteht, die jeweils einen Innenkanal aufweisen, dessen eines Ende mit dem Behälter (66) in Verbindung steht und dessen anderes Ende eine Durchgangsöffnung aufweist.

**Revendications**

1. Cartouche (30, 60, 110) pour l'emmagasinage d'une dose d'un produit pharmaceutique (38, 70, 122) sous forme sèche, ainsi que pour une utilisation éventuelle avec une trousse d'administration par voie intraveineuse (14) dans le but de faciliter l'infusion, par voie intraveineuse, de la dose en solution dans un liquide approprié (12), la trousse d'administration par voie intraveineuse (14) comportant une alimentation du liquide (12) qui y est raccordée au cours de l'utilisation, ainsi qu'un moyen de raccordement (18, 98, 100, 140) destiné à être utilisé pour former un raccord de communication pour un liquide entre la cartouche et la trousse d'administration par voie intraveineuse, cette cartouche comprenant:
un récipient étanche aux liquides (34, 66, 118) destiné à contenir la dose du produit pharmaceutique sec (38, 70, 122);
un moyen de raccordement (32, 62, 64, 112) communiquant avec le récipient et conçu pour correspondre avec le moyen de raccordement (18, 98, 100, 140) de la trousse d'administration par voie intraveineuse (14) en vue de former un raccord de communication pour un liquide entre cette dernière et le récipient (34, 66, 118);
caractérisée en ce que le récipient comporte intérieurement un moyen d'arrêt (42, 72, 124) destiné à empêcher la sortie, hors du récipient (34, 66, 118), de particules solides d'une granularité supérieure à une valeur prédéterminée qui pourraient provoquer virtuellement une irritation des veines, ce moyen d'arrêt (42, 72, 124) étant perméable aux liquides et aux solutions, de telle sorte que le liquide provenant de la trousse d'administration par voie intraveineuse (14) puisse entrer en contact avec et dissoudre le produit pharmaceutique (38, 70, 122), tout en permettant, à la solution du produit pharmaceutique ainsi obtenue, de passer à travers le moyen d'arrêt (42, 72, 124) et à travers le raccord de communication à la trousse d'administration par voie intraveineuse (14) en vue de l'infusion; et
le récipient (34, 66, 118) est pourvu d'un évent (44, 76, 80, 130) en vue de faciliter le passage du liquide dans le récipient à partir de la trousse d'administration par voie intraveineuse, cet évent comprenant un élément d'arrêt liquide perméable à l'air (48, 78, 82, 134) destiné à empêcher l'échappement du liquide au travers.

2. Cartouche (39, 60, 110) selon la revendication 1, comprenant une dose de produit pharmaceutique sec (38, 70, 122) disposée dans le récipient (34, 66, 118).

3. Cartouche (30, 60, 110) selon la revendication 1, destinée à une utilisation avec une trousse d'administration par voie intraveineuse (14) comportant un moyen de raccordement (18, 98, 100, 140) comprenant un obturateur en matière élastomère (18a, 98, 100, 148), caractérisée en ce que le moyen de raccordement (32, 62, 64, 112) comprend un conduit comportant un canal intérieur qui communique avec le récipient à une extrémité et débouche dans un orifice à l'extrémité distale du conduit, cette extrémité distale du conduit étant conçue pour pénétrer dans l'obturateur de la trousse d'administration en vue de former le raccord de communication.

4. Cartouche (110) selon la revendication 1, caractérisée en ce que son moyen de raccordement (112) comprend un conduit (112) comportant un premier canal intérieur (114) qui communique avec le récipient (118) à une extrémité et débouche dans un premier orifice à l'extrémité distale du conduit en vue de véhiculer le liquide de la trousse d'administration par voie intraveineuse au récipient, ainsi qu'un second canal intérieur séparé (116) qui communique avec le récipient (118) à une extrémité et débouche dans un second orifice à l'extrémité distale du conduit en vie de véhiculer la solution de produit pharmaceutique (122) du récipient (118) à la trousse d'administration par voie intraveineuse, cette cartouche (110) étant destinée à être utilisée avec une trousse d'administration par voie intraveineuse comportant un moyen de raccordement (140) conçu pour diriger le liquide de la trousse d'administration par voie intraveineuse au premier orifice, ainsi que pour recevoir la solution provenant du second orifice après le passage du liquide à travers le récipient.

5. Cartouche selon la revendication 1, caractérisée en ce que le moyen de raccordement (62, 64) de la cartouche (60) comprend plusieurs conduits (62, 64) comportant chacun un canal intérieur qui communique avec le récipient (66) à une extrémité et débouche dans un orifice à l'extrémité distale du conduit.

FIG.1

FIG.2

FIG.3

0 077 604

FIG. 6

FIG. 5

FIG. 4

2